(19) 

Europäisches
Patentamt
European
Patent Office
Office européen
des brevets

(11) **EP 3 741 457 A1**

(12) **EUROPEAN PATENT APPLICATION**
published in accordance with Art. 153(4) EPC

(43) Date of publication:
**25.11.2020 Bulletin 2020/48**

(21) Application number: **19741542.5**

(22) Date of filing: **17.01.2019**

(51) Int Cl.:
**B01J 23/80** *(2006.01)* **C07C 1/24** *(2006.01)*
**C07C 11/167** *(2006.01)* **C07B 61/00** *(2006.01)*
**C07C 11/04** *(2006.01)* **C07C 11/06** *(2006.01)*
**C07C 45/29** *(2006.01)* **C07C 47/07** *(2006.01)*

(86) International application number:
**PCT/JP2019/001294**

(87) International publication number:
**WO 2019/142865 (25.07.2019 Gazette 2019/30)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **17.01.2018 JP 2018005943**

(71) Applicant: **SEKISUI CHEMICAL CO., LTD.
Osaka-shi
Osaka
530-8565 (JP)**

(72) Inventors:
• **YAGIHASHI Noritoshi**
**Tsukuba-shi, Ibaraki 300-4292 (JP)**
• **MIYAMA Toshihito**
**Tsukuba-shi, Ibaraki 300-4292 (JP)**
• **NISHIYAMA Haruka**
**Tsukuba-shi, Ibaraki 300-4292 (JP)**

(74) Representative: **Ter Meer Steinmeister & Partner
Patentanwälte mbB
Nymphenburger Straße 4
80335 München (DE)**

(54) **CATALYST AND METHOD FOR PRODUCING DIENE COMPOUND USING SAID CATALYST**

(57) The present invention relates to a catalyst comprising, as catalyst components: a compound comprising at least one element X selected from the group consisting of elements belonging to Groups 3 to 6 of the periodic table; a zinc compound; and a compound comprising at least one element Y selected from the group consisting of elements belonging to Groups 7 to 11 of the periodic table, and wherein the catalyst has an average pore diameter of 2 to 50 nm.

EP 3 741 457 A1

**Description**

[Technical Field]

The present invention relates to a catalyst and a method for producing a diene compound using the catalyst.
Priority is claimed on Japanese Patent Application No. 2018-005943, filed on January 17, 2018, the content of which is incorporated herein by reference.

[Background Art]

Butadiene such as 1,3-butadiene, which is a representative example of diene compounds, is used as a raw material for styrene-butadiene rubber (SBR) and the like. Conventionally, butadiene has been purified from the C4 fraction. The C4 fraction is a fraction obtained as a by-product during naphtha cracking for producing ethylene from petroleum. However, the use of petroleum has decreased as a result of increased use of shale gas. Consequently, the butadiene production by naphtha cracking of petroleum has also decreased. Therefore, there is a need for an alternative method for producing 1,3-butadiene.
For example, Patent Document 1 discloses an invention relating to a metal-impregnated silica catalyst for selectively converting ethanol into butadiene. More specifically, Patent Document 1 describes a butadiene synthesis catalyst including Hf and two or more catalytically active metals M1 and M2, wherein the two or more catalytically active metals M1 and M2 are selected from the group consisting of Zr, Zn, Cu, and combinations thereof, provided that M1 and M2 are different.
Patent Document 1 describes a method for synthesizing butadiene, which includes (i) providing a gas stream G-1 containing ethanol and optional acetaldehyde, and (ii) contacting the gas stream G-1 with the butadiene synthesis catalyst to obtain a gas stream G-2 containing butadiene.
Patent Document 1 describes that the method for synthesizing butadiene achieves a butadiene selectivity of at least 10 %.

[Related Literature]

[Patent Literature]

[Patent Document 1]
International Patent Application Publication No. 2014/199349

[Summary of Invention]

[Technical Problem]

Although the invention described in Patent Document 1 enables butadiene synthesis using ethanol as a raw material gas, it is necessary to reduce the ethanol concentration in the raw material gas. For this reason, according to the invention described in Patent Document 1, the yield of butadiene per unit time is low, and it is difficult to increase the production efficiency of butadiene.
In this situation, the object of the present invention is to provide a catalyst that can efficiently produce a diene compound even when the alcohol concentration in the raw material gas is high.

[Solution to Problem]

The present inventors have made intensively studies to solve the above-mentioned problems. As a result, they have found that the above problems can be solved by a specific catalyst having mesopores, and have completed the present invention. In other words, the present invention includes the following embodiments.

[1] A catalyst including, as catalyst components: a compound containing at least one element X selected from the group consisting of elements belonging to Groups 3 to 6 of the periodic table; a zinc (Zn) compound; and a compound containing at least one element Y selected from the group consisting of elements belonging to Groups 7 to 11 of the periodic table, in which the catalyst has an average pore diameter of 2 to 50 nm.
[2] The catalyst according to [1], in which the catalyst components are supported on a porous carrier.
[3] The catalyst according to [2], in which the porous carrier has mesopores.
[4] The catalyst according to any one of [1] to [3], in which a specific surface area of the catalyst is 50 to 1000 $m^2/g$.

[5] The catalyst according to any one of [1] to [4], which is a catalyst for synthesizing a diene compound from a raw material gas containing an alcohol.

[6] The catalyst according to [5], in which the raw material gas includes ethanol, or a mixture of ethanol and acetaldehyde.

[7] A method for producing a diene compound, including contacting the catalyst of any one of [1] to [6] with a raw material gas containing an alcohol to obtain a diene compound.

[8] The method according to [7], in which an amount of the alcohol is 10 % by volume or more with respect to 100 % by volume of the raw material gas.

[Advantageous Effects of Invention]

The catalyst of the present invention enables efficient production of a diene compound even when the alcohol concentration in the raw material gas is high.

[Brief Description of Drawings]

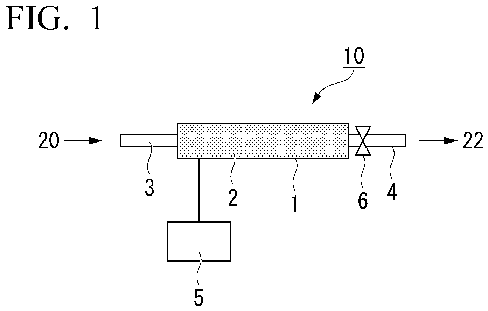

FIG. 1 is a schematic view of an apparatus for producing butadiene according to one embodiment of the present invention.

[Description of Embodiments]

Hereinbelow, embodiments of the present invention are described in detail, but the following descriptions illustrate only examples of the embodiments of the present invention, and the present invention is not limited thereto and may be modified as long as the modifications do not deviate from the gist of the present invention.

(Catalyst)

The catalyst of the present invention includes, as catalyst components, a compound (hereinafter, simply referred to as "compound containing element X") containing at least one element X selected from the group consisting of elements belonging to Groups 3 to 6 of the periodic table, a zinc (Zn) compound, and a compound (hereinafter, simply referred to as "compound containing element Y") containing at least one element Y selected from the group consisting of elements belonging to Groups 7 to 11 of the periodic table. The catalyst has an average pore diameter of 2 to 50 nm.

By virtue of including the zinc (Zn) compound and the compound containing element Y, the alcohol can be converted into the aldehyde. In addition, by virtue of including the compound containing element X and the zinc (Zn) compound, the alcohol and the aldehyde can be converted to the diene compound. Furthermore, by virtue of combining the compound containing element X and the zinc (Zn) compound, and the compound containing element Y, the diene compound can be efficiently produced even when the alcohol concentration in the raw material gas is high.

The compound containing element X is not particularly limited. Examples thereof include oxides; inorganic salts such as, chlorides, sulfides, nitrates, and carbonates; organic salts or chelate compounds such as oxalate, acetylacetonate, dimethylglyoxime salt, and ethylenediamine acetate; carbonyl compounds; cyclopentadienyl compounds; ammine complexes; alkoxide compounds; alkyl compounds; and the like. Among these, the compound containing element X preferably contains oxides containing element X from the viewpoint of increasing the production efficiency of the diene compound.

Examples of element X include Group 3 elements such as scandium (Sc), yttrium (Y), lanthanum (La), and cerium (Ce); Group 4 elements such as titanium (Ti), zirconium (Zr), and hafnium (Hf); Group 5 elements such as vanadium (V), niobium (Nb), and tantalum (Ta); and Group 6 elements such as chromium (Cr), molybdenum (Mo), and tungsten (W). Among them, yttrium (Y), lanthanum (La), cerium (Ce), titanium (Ti), zirconium (Zr), hafnium (Hf), vanadium (V), niobium (Nb), tantalum (Ta), molybdenum (Mo) and tungsten (W) are preferable; yttrium (Y), lanthanum (La), cerium (Ce), titanium (Ti), zirconium (Zr), hafnium (Hf), niobium (Nb) and tantalum (Ta) are more preferable; zirconium (Zr), hafnium (Hf), niobium (Nb) and tantalum (Ta) are even more preferable; zirconium (Zr), hafnium (Hf) and niobium (Nb) are even more preferable; zirconium (Zr) and hafnium (Hf) are particularly preferable; and hafnium (Hf) is most preferable.

Specific compounds containing element X include, but are not particularly limited, hafnium oxide ($HfO_2$, $Hf_2O_5$), zirconium oxide ($ZrO_2$, $Zr_2O_5$), niobium oxide ($NbO_2$, $Nb_2O_5$), tantalum oxide ($TaO_2$, $Ta_2O_5$), titanium chloride ($TiCl_2$, $TiCl_3$, $TiCl_4$), zirconium chloride ($ZrCl_2$), hafnium chloride ($HfCl_4$), niobium chloride ($NbCl_5$), tantalum chloride ($TaCl_5$), vanadium chloride ($VCl_3$), tungsten chloride ($WCl_5$), scandium nitrate ($Sc(NO_3)_3$), yttrium nitrate ($Y(NO_3)_3$), lanthanum nitrate ($La(NO_3)_3$), and cerium nitrate ($Ce(NO_3)_3$).

Among these, the compound containing element X preferably contains hafnium oxide, zirconium oxide, niobium oxide, and tantalum oxide, more preferably hafnium oxide, zirconium oxide, and niobium oxide, even more preferably hafnium oxide, zirconium oxide, and particularly preferably hafnium oxide.

One of the compounds containing element X described above may be used alone, or two or more thereof may be used in combination.

The zinc (Zn) compound is a compound containing zinc element. The zinc (Zn) compound is not particularly limited. Examples thereof include oxides; inorganic salts such as, chlorides, sulfides, nitrates, and carbonates; organic salts or chelate compounds such as oxalate, acetylacetonate, dimethylglyoxime salt, and ethylenediamine acetate; carbonyl compounds; cyclopentadienyl compounds; ammine complexes; alkoxide compounds; alkyl compounds; and the like.

Specific compounds containing zinc element include, but are not particularly limited, zinc oxide (ZnO), zinc chloride ($ZnCl_2$), zinc sulfide (ZnS), zinc nitrate ($Zn(NO_3)_2$) and the like. Among these, the compound containing zinc element is preferably containing zinc oxide (ZnO) from the viewpoint of increasing the production efficiency of the diene compound.

One of the compounds containing zinc element described above may be used alone, or two or more thereof may be used in combination.

The compound containing element Y is not particularly limited. Examples thereof include oxides; inorganic salts such as, chlorides, sulfides, nitrates, and carbonates; organic salts or chelate compounds such as oxalate, acetylacetonate, dimethylglyoxime salt, and ethylenediamine acetate; carbonyl compounds; cyclopentadienyl compounds; ammine complexes; alkoxide compounds; alkyl compounds; and the like. Among these, the compound containing element Y preferably contains oxides containing element Y from the viewpoint of increasing the production efficiency of the diene compound.

Examples of element Y include Group 7 elements such as manganese (Mn), technetium (Tc), and rhenium (Re); Group 8 elements such as iron (Fe) and ruthenium (Ru); Group 9 elements such as cobalt (Co), rhodium (Rh), and iridium (Ir) ; Group 10 elements such as nickel (Ni), palladium (Pd), and platinum (Pt); and Group 11 elements such as copper (Cu), silver (Ag), and gold (Au). Among them, manganese (Mn), iron (Fe), cobalt (Co), rhodium (Rh), nickel (Ni), palladium (Pd), platinum (Pt), copper (Cu), silver (Ag), and gold (Au) are preferable; manganese (Mn), iron (Fe), cobalt (Co), nickel (Ni), and copper (Cu) are more preferable; nickel (Ni) and copper (Cu) are even more preferable; and copper (Cu) is particularly preferable.

Specific compounds containing element Y include, but are not particularly limited, manganese oxide (MnO, $Mn_3O_4$, $Mn_2O_3$, $MnO_2$, $MnO_3$, $Mn_2O_7$), iron oxides (FeO, $Fe_3O_4$, $Fe_2O_3$), cobalt oxide (CoO, $CO_2O_3$, $Co_3O_4$), rhodium oxide ($RhO_2$, $Rh_2O_3$), nickel oxide (NiO, $NiO_2$, $Ni_2O3$), palladium oxide (PdO, $PdO_2$), platinum oxide (PtO, $PtO_2$), copper oxide (CuO, $Cu_2O$), silver oxide (AgO, $Ag_2O$), gold oxide ($Au_2O_3$), iron chloride ($FeCl_2$, $FeCl_3$), nickel chloride ($NiCl_2$), palladium chloride ($PdCl_2$), copper chloride (CuCl, $CuCl_2$), silver chloride (AgCl), iron nitrate ($Fe(NO_3)_2$, $Fe(NO_3)_3$), nickel nitrate ($Ni(NO_3)_2$), palladium nitrate ($Pd(NO_3)_2$), copper nitrate ($Cu(NO_3)_2$), silver nitrate ($AgNO_3$), and the like.

Among these, the compound containing element Y preferably contains manganese oxide, iron oxide, cobalt oxide, rhodium oxide, nickel oxide, palladium oxide, platinum oxide, copper oxide, silver oxide, and gold oxide, more preferably manganese oxide, iron oxide, cobalt oxide, nickel oxide, and copper oxide, even more preferably nickel oxide and copper oxide, and particularly preferably copper oxide.

One of the compounds containing element Y described above may be used alone, or two or more thereof may be used in combination.

As a combination of the compound containing element X, the zinc compound, and the compound containing element Y, hafnium compound-zinc compound-copper compound, zirconium compound-zinc compound-copper compound, niobium compound-zinc compound-copper compound, hafnium compound-zinc compound-nickel compound, and zirconium compound-zinc compound-nickel compound are preferable, hafnium compound-zinc compound-copper compound, hafnium compound-zinc compound-nickel compound, and zirconium compound-zinc compound-copper compound are more preferable, and hafnium compound-zinc compound-copper compound and zirconium compound-zinc compound-copper compound are even more preferable.

The amount of element X in the catalyst components is preferably 2.0 to 65% by mass, more preferably 30 to 60% by mass, with respect to the total mass of the catalyst components. When the amount of element X is 2.0% by mass or more, a sufficient amount of catalyst component X is included and the conversion of the raw material and the selectivity for the diene compound are further increased, which is preferable. On the other hand, when the amount of element X is 65% by mass or less, the catalyst component X is likely to be uniformly dispersed, and the selectivity for the diene compound is further increased, which is preferable.

The amount of zinc element in the catalyst components is preferably 0.1 to 40% by mass, more preferably 5 to 25% by mass, with respect to the total mass of the catalyst components. When the amount of zinc element is 0.1% by mass or more, a sufficient amount of zinc element is included and the conversion of the raw material and the selectivity for the diene compound are further increased, which is preferable. On the other hand, when the amount of zinc element is 40% by mass or less, zinc element is likely to be uniformly dispersed, and the selectivity for the diene compound is further increased, which is preferable.

The amount of element Y in the catalyst components is preferably 0.1 to 55% by mass, more preferably 1.0 to 25% by mass, with respect to the total mass of the catalyst components. When the amount of element Y is 0.1% by mass or more, a sufficient amount of catalyst component Y is included and the conversion of the raw material and the selectivity for the diene compound are further increased, which is preferable. On the other hand, when the amount of element Y is 55% by mass or less, the catalyst component Y is likely to be uniformly dispersed, and the selectivity for the diene compound is further increased, which is preferable.

In the present specification, the amount (mass) of each element in the catalyst components can be measured by high-frequency inductively coupled plasma emission spectroscopy.

The mass ratio (element X/Zn) of element X to zinc element in the catalyst components is preferably 2/1 to 15/1 and more preferably 4/1 to 8/1. The mass ratio of not less than 2/1 is preferable in that the conversion of the raw material is further increased. On the other hand, the mass ratio of not more than 15/1 is preferable in that the selectivity for the diene compound is further increased. The "conversion of the raw material" means a percentage of the number of moles consumed in the reaction out of the number of moles of the raw material contained in the raw material gas. The "selectivity for the diene compound" means a percentage of the number of moles of the raw material converted to the diene compound out of the number of moles of the raw material consumed in the reaction using the catalyst. When only the alcohol is used as the raw material, the number of moles of the raw material is the number of moles of the alcohol. When both the alcohol and the aldehyde are used as the raw materials, the number of moles of the raw materials is the total number of moles thereof.

The mass ratio of element Y to element X (element Y/element X) in the catalyst components is preferably 1/6 to 1/1 and more preferably 1/5 to 1/2. The mass ratio of not less than 1/6 is preferable in that the selectivity for the diene compound is further increased. On the other hand, the mass ratio of not more than 1/1 is preferable in that the conversion of the raw material is further increased.

The mass ratio of element Y to zinc element (element X/Zn) in the catalyst components is preferably 1/5 to 5/1 and more preferably 1/3 to 3/1. The mass ratio of not less than 1/5 is preferable in that the conversion of the raw material is further increased. On the other hand, the mass ratio of not more than 5/1 is preferable in that the selectivity for the diene compound is further increased.

The form of the catalytic components in the catalyst is not particularly restricted, but the compound containing element X, the zinc (Zn) compound, and the compound containing element Y may be included in an independent form, or it may be included as a complex oxide containing two or more elements selected from the group consisting of element X, zinc element, and element Y. The term "complex oxide" refers to a complex of two or more oxides.

The form of the catalytic components in the catalyst may be an aggregate or a condensate.

The catalyst may be a supported catalyst in which the catalyst components are supported on a porous carrier.

As the porous carrier, well-known porous carrier can be used. Examples thereof include silica, titania, alumina, and the like. Among them, silica is preferable as a porous carrier. There are various silica products with different specific surface areas and pore diameters. By combining the specific surface area and pore diameter of the porous carrier, the selectivity for the diene compound and the conversion of the material gas can be controlled.

Among the porous carriers, it is preferable to use porous carrier with mesopores, and it is more preferable to use mesoporous silica (hereinafter referred to as "mesoporous silica"). In the present specification, the term "having mesopores" means an average pore diameter of 2 to 50 nm.

Conventionally known mesoporous silica can be used as the mesoporous silica.

The amount of the catalyst components supported on the porous carrier is preferably 1 to 30 % by mass, more preferably 2.5 to 10 % by mass, with respect to 100 % by mass of the porous carrier. When the amount of the supported catalyst components is not less than the lower limit described above, a sufficient amount of the catalyst components can be supported, and the conversion of the raw material and the selectivity for the diene compound can be further increased. When the amount of the supported catalyst components is not more than the upper limit described above, the catalyst components can be uniformly and highly dispersed with ease, so that the selectivity for the diene compound can be further increased.

The amount of element X supported on the porous carrier is preferably 0.5 to 10 % by mass, more preferably 1 to 5 % by mass, even more preferably 2 to 4 %, in terms of element, with respect to 100 % by mass of the porous carrier.

The amount of Zn supported on the porous carrier is preferably 0.1 to 5 % by mass, more preferably 0.2 to 2 % by mass, even more preferably 0.3 to 1 %, in terms of element, with respect to 100 % by mass of the porous carrier.

The amount of element Y supported on the porous carrier is preferably 0.1 to 8 % by mass, more preferably 0.3 to 5 % by mass, even more preferably 0.5 to 2 %, in terms of element, with respect to 100 % by mass of the porous carrier.

The catalyst preferably has a composition satisfying formula (I):

$$X_x Zn_y Y_z \qquad (I)$$

In formula (I), x, y and z are numbers representing the mass ratio of element X, Zn and element Y, respectively, and x is 0.5 to 10, y is 0.1 to 5 and z is 0.1 to 8.

In the formula (I), x is preferably 0.5 to 10, more preferably 1 to 5, and even more preferably 2 to 4. When x is within the above range, the selectivity for the diene compound can be further increased.

In the formula (I), y is preferably 0.1 to 5, more preferably 0.2 to 2, and even more preferably 0.3 to 1. When y is within the above range, the selectivity for the diene compound can be further increased.

In the formula (I), z is preferably 0.1 to 8, more preferably 0.3 to 5, and even more preferably 0.5 to 2. When z is within the above range, the selectivity for the diene compound can be further increased

The average particle size of the catalyst is not particularly limited, preferably 150 to 3000 $\mu$m, more preferably 200 to 1500 $\mu$m, and even more preferably 300 to 1000 $\mu$m. The average particle size of the catalyst can be controlled by sifting. When the average particle size is within the range described above, pressure loss can be suppressed. The catalyst is preferably one with a particle size distribution as narrow as possible. In the present specification, "particle size" means the maximum length of the distance between two points on the contour of a particle. The value of "average particle diameter" is the average value of 200 arbitrary particle diameters within a single field of view obtained by an optical microscope.

The total of the pore volumes of the catalyst (hereinafter, also referred to as the total pore volume) is preferably 0.1 to 2.50 mL/g, more preferably 0.25 to 1.50 mL/g, and even more preferably 0.50 to 1.20 mL/g. When the total pore volume is not less than the lower limit described above, the diffusivity of the raw material containing the alcohol inside the catalyst is improved, and the conversion of the raw material and the selectivity for the diene compound are further increased. When the total pore volume is not more than the upper limit described above, a sufficient contact area between the raw material and the catalyst is likely to be achieved, and the conversion of the raw material and the selectivity for the diene compound are further increased. In the present specification, the "total pore volume" of the catalyst is a value measured by a gas adsorption method. The gas adsorption method is a method in which nitrogen gas is used as an adsorption gas, nitrogen molecules are allowed to be adsorbed on the surface of the catalyst, and pore distribution is measured from condensation of the molecules.

The average pore diameter of the catalyst is 2 to 50 nm, preferably 2 to 17.5 nm, more preferably 7 to 17.5 nm, and even more preferably 10 to 15 nm. In other words, the catalyst has mesopores. When the average pore diameter is not less than the lower limit described above, the diffusivity of the raw material containing the alcohol inside the catalyst is improved, and the conversion of the raw material is further increased. Also, when the average pore diameter is not less than the lower limit described above, the acid strength of the catalyst does not become too strong, and side reactions can be suppressed, resulting in a higher selectivity for the diene compounds. When the average diameter is not more than the upper limit described above, a sufficient amount of active sites are provided on the catalyst surface, which allows for further increase in the conversion of the raw material and the selectivity for the diene compound.

The average pore diameter is a value calculated from the total pore volume (total of the pore volumes of the catalyst) and the BET specific surface area. The BET specific surface area is a value calculated from an adsorbed amount of nitrogen that is an adsorption gas, and a pressure at the time of adsorption. Specifically, the average pore diameter can be calculated on the assumption that the pores are in the form of a cylinder. With the BET specific surface area A1 being used as the side area of the cylinder and the total pore volume VI being used as the volume of the cylinder, the average pore diameter Davel can be calculated by formula (II) below.

$$D_{ave1} = 4V1/A1 \qquad (II)$$

The specific surface area of the catalyst is preferably 50 to 1000 $m^2/g$, more preferably 200 to 500 $m^2/g$, even more preferably 200 to 300 $m^2/g$, and particularly preferably 250 to 300 $m^2/g$. When the specific surface area is not less than the lower limit described above, a sufficient amount of active sites are provided on the catalyst surface, which allows for further increase in the conversion of the raw material and the selectivity for the diene compound. When the specific surface area is not more than the upper limit described above, the average pore diameter does not become too small, the diffusivity of the raw material containing the alcohol inside the catalyst is improved, and the conversion of the raw material is further increased. The specific surface area means the BET specific surface area, which is measured by the BET gas adsorption method using nitrogen as an adsorption gas.

The product of the total pore volume times the specific surface area of the catalyst is preferably 5 to 2500 mL·$m^2/g^2$, and more preferably 100 to 500 mL·$m^2/g^2$. When the product is not less than the lower limit described above, a sufficient amount of active sites are provided on the catalyst surface, which allows for further increase in the conversion of the raw material and the selectivity for the diene compound. When the product is not more than the upper limit described above, the surface of the catalyst which does not serve as an active site, does not become too large, and side reactions can be suppressed, resulting in a higher selectivity for the diene compound.

The total pore volume of the mesopore of the catalyst (hereinafter referred to as the total mesopore volume) is preferably 50 to 100 %, more preferably 80 to 100%, and even more preferably 90 to 100%, with respect to the total pore volume of the catalyst. When the total mesopore volume to the total pore volume is not less than the lower limit described above, a sufficient amount of mesopores are provided inside the catalyst, the diffusivity of the raw material containing the alcohol inside the catalyst is improved, and the conversion of the raw material and the selectivity for the diene compound are further increased.

The total pore volume is a value measured by the aforementioned gas adsorption method. The total mesopore volume of the catalyst can be obtained by measuring the volume of the pores having 2-50 nm by nitrogen adsorption method and summing them.

[Method for producing catalyst]

The method of producing the catalyst is not particularly limited, and can be produced by any known method. For example, when the catalyst consists of catalyst components, it can be produced by mixing a compound containing element X, a zinc compound, and a compound containing element Y with an organic polymer, and solidifying, and calcining. The loss of the organic polymer by calcination allows the production of catalysts (with mesopores) with an average pore diameter of 2 to 50 nm.

The compound containing element X, the zinc compound, and the compound containing element Y may be transformed before and after calcination, depending on the type of compound used. For example, when hafnium chloride is used as a compound containing element X, all of the hafnium chloride can be converted to hafnium oxide by calcination. In this case, the compound containing element X in the catalyst components is hafnium oxide. When hafnium chloride is used as a compound containing element X, a part of hafnium chloride can be converted to hafnium oxide by calcination. In this case, the compound containing element X is hafnium oxide and hafnium chloride in the catalyst components.

Examples of the organic polymer include polystyrene, polyacrylic acid, polymethacrylic acid, polyethylene, polypropylene, polyacrylic acid ester, polymethacrylic acid ester, and copolymers of monomers constituting the aforementioned organic polymer. One of these organic polymers may be used alone, or two or more thereof may be used in combination.

The average pore diameter can be controlled by controlling the type of the organic polymer, particle size, calcination temperature, and the like.

When the catalyst includes catalyst components and a porous carrier, the catalyst can be obtained by supporting the compound containing element X, the zinc compound, and the compound containing element Y on the porous carrier, and then drying and calcination.

The method of obtaining the porous carrier is not particularly limited, and may be used one produced by conventional manufacturing methods or commercial products.

The method for producing mesoporous silica is, for example, the sol-gel method by mixing a compound containing silicon element with a solution containing an inorganic acid. Examples of the compound containing silicon element include sodium silicate, potassium silicate, ammonium silicate, and the like. Examples of the inorganic acid include sulfuric acid, nitric acid, hydrochloric acid, and the like.

Examples of the commercial products of mesoporous silica include CARiACT Q series manufactured by Fuji Silysia chemical LTD. such as Q-6 (average pore diameter: 5.7 nm, total pore volume: 0.61 mL/g, specific surface area: 430 $m^2$/g), Q-10 (average pore diameter: 12.7 nm, total pore volume: 0.95 mL/g, specific surface area: 300 $m^2$/g), Q-15 (average pore diameter: 20.3 nm, total pore volume: 0.99 mL/g, specific surface area: 195 $m^2$/g), Q-30 (average pore diameter: 36.6 nm, total pore volume: 0.98 mL/g, specific surface area: 107 $m^2$/g).

Examples of the raw material compound include oxides; inorganic salts such as chlorides, sulfides, nitrates and carbonates; organic salts or chelate compounds such as oxalates, acetylacetonate salts, dimethylglyoxime salts and ethylenediamine acetic acid salts; carbonyl compounds; cyclopentadienyl compounds; amine complexes; alkoxide compounds; and alkyl compounds.

The method of supporting the catalyst components on the porous carrier is the same as the method of supporting the catalyst components on the carrier as previously known, except that the porous carrier is used as a carrier and the compound containing element X, the Zn compound, and the compound containing element Y are used as the catalyst components. Examples of the method of supporting the catalyst components on to the carrier as previously known include impregnation method, coprecipitation method, ion exchange method, and the like.

Examples of the impregnation method include: the evaporation drying method in which the catalyst components are allowed to be supported on the porous carrier by immersing the porous carrier in an impregnation solution used in an excess amount relative to the total pore volume of the porous carrier, followed by drying; the equilibrium adsorption method in which the catalyst components are allowed to be supported on the porous carrier by implementing solid-liquid separation such as filtration after immersing the porous carrier in an impregnation solution used in an excess amount relative to the total pore volume of the porous carrier; and the pore filling method in which the catalyst components are allowed to be supported on the porous carrier by immersing the porous carrier in an impregnation solution used in an amount approximately equal to the total pore volume of the porous carrier, followed by drying.

The pore filling method is preferable to have a predetermined amount of the catalyst components supported on the porous carrier homogeneously and with high degree of dispersion.

The total pore volume of the porous carrier can be measured in the same manner as in the case of measurement of the total pore volume of the catalyst.

The pore filling ratio in the pore filling method is usually 80 to 120%, preferably 90 to 110%, and more preferably 95 to 105%.

The pore filling ratio is a ratio of the volume of impregnation solution used for impregnation with respect to the total pore volume of the porous carrier.

A predetermined supported amount of catalyst components and a desirable range of the pore filling ratio described above can be satisfied by adjusting the volume of the impregnation solution in which the raw material compounds of the catalyst components are dissolved in a solvent. Examples of the solvent include water, methanol, ethanol, tetrahydrofuran, dioxane, hexane, benzene, and toluene.

Examples of the method for impregnating the porous carrier with the impregnation solution include a simultaneous method, a sequential method and the like. The simultaneous method is a method of impregnating a carrier with a solution in which all the raw material compounds are dissolved. The sequential method is a method in which respective solutions of the raw material compounds dissolved are separately prepared, and a carrier is sequentially impregnated with the solutions.

Examples of the sequential method include a method of obtaining a catalyst precursor by impregnating a porous carrier with a first impregnation solution in which a raw material compound containing the element X has been dissolved, and subsequently impregnating the catalyst precursor with a second impregnation solution in which a raw material compound containing Zn and a raw material compound containing the element Y have been dissolved.

The drying temperature and the calcination temperature may be appropriately determined depending on the type of the solvent and the like. For example, when the solvent is water, the drying temperature may be 80 to 120 °C, and the calcination temperature may be 300 to 600 °C.

In the case of the sequential method described above, the impregnation with the second impregnation solution may be implemented after drying of the product impregnated with the first impregnation solution, or after drying and subsequent calcination of the product impregnated with the first impregnation solution.

When the catalyst is produced using the porous carrier as described above, the average pore diameter of the catalyst can be easily controlled by selecting the appropriate porous carrier and catalyst production conditions such that the average pore diameter of the porous carrier is maintained in the catalyst.

(Apparatus for producing diene compound)

The apparatus for producing a diene compound includes a reaction tube filled with the catalyst described above. The apparatus of the present invention produces a diene compound from a raw material gas containing a raw material.

Hereinbelow, a butadiene production apparatus, which is one type of the apparatus for producing a diene compound, is described with reference to FIG. 1.

The butadiene production apparatus 10 of the present embodiment (hereinafter, simply referred to as "production apparatus 10") includes a reaction tube 1, a supply pipe 3, an outlet pipe 4, a temperature controller 5, and a pressure controller 6.

The reaction tube 1 has a reaction bed 2 inside. The reaction bed 2 is packed with the catalyst of the present invention. The supply pipe 3 is connected to the reaction tube 1. The outlet pipe 4 is connected to the reaction tube 1. The temperature controller 5 is connected to the reaction tube 1. The outlet pipe 4 is equipped with the pressure controller 6.

The reaction bed 2 may have only the catalyst of the present invention, or may have a diluent as well as the catalyst of the present invention. The diluent prevents the catalyst from generating excessive heat.

Here, the reaction for synthesizing butadiene from the raw material is an endothermic reaction. For this reason, the reaction bed 2 usually does not require a diluent.

The diluent may be, for example, the same as in the carrier of the synthetic catalyst, quartz sand, alumina balls, aluminum balls, aluminum shots, and the like.

When a diluent is charged into the reaction bed 2, the mass ratio, diluent/catalyst, is determined in consideration of the type, specific gravity and the like of the diluent and the catalyst, and is, for example, preferably 0.5 to 5.

The reaction bed may be any of a fixed bed, a moving bed, a fluidized bed, and the like.

The reaction tube 1 is preferably made of a material that is inert to the raw material gas and the synthesized product. The reaction tube 1 preferably has a shape that enables the reaction tube 1 to withstand heating at about 100 to 500 °C or pressurization at about 10 MPa. The reaction tube 1 may be, for example, a substantially cylindrical member made of stainless steel.

The supply pipe 3 is a supply means that supplies the raw material gas into the reaction pipe 1. The supply pipe 3 is, for example, a pipe made of stainless steel.

The outlet pipe 4 is an outlet means that releases a gas containing a product synthesized in the reaction bed 2. The outlet pipe 4 is, for example, a pipe made of stainless steel or the like.

With respect to the temperature controller 5, there is no particular limitation as long as it can control the temperature of the reaction bed 2 in the reaction tube 1 to a desired value. For example, the temperature controller 5 may be an electric furnace or the like.

With respect to the pressure controller 6, there is no particular limitation as long as it can control the internal pressure of the reaction tube 1 to a desired value. For example, the pressure controller 6 may be a known pressure valve or the like.

The production apparatus 10 may be equipped with a known device such as a gas flow rate controller (e.g., mass flow controller) or the like which adjusts a flow rate of the gas.

<Method for producing diene compound>

The method for producing a diene compound includes contacting a raw material gas containing an alcohol with the catalyst of the present invention to thereby obtain a diene compound.

< Catalyst >

The catalyst to be used is as described above and therefore detailed descriptions are omitted here.

The amount of the catalyst to be used is preferably 0.1 to 10 g/g-h, and more preferably 1 to 5 g/g-h, based on the amount of the raw material gas. The catalyst amount of not less than 0.1 g/g·h is preferable in that the reaction conversion can be improved. On the other hand, the catalyst amount of not more than 10 g/g·h is preferable in that generation of by-products can be suppressed.

<Raw material gas>

The raw material gas contains an alcohol. In addition, the raw material gas may further include an aldehyde, an inert gas, and the like.

(Alcohol)

Examples of the alcohol include, but are not particularly limited, alcohols having 1 to 6 carbon atoms. Specific examples of the alcohol include methanol, ethanol, propanol, butanol, pentanol, hexanol and the like.

In principle, the diene compound to be obtained depends on the type of alcohol used. For example, when ethanol is used, butadiene is obtained. When propanol is used, hexadiene is obtained. When butanol is used, octadiene is obtained.

The alcohol used may be of a single type or a mixture of two or more types, but is preferably of a single type from the viewpoint of suppressing side reactions.

The concentration of the alcohol in the raw material gas is preferably 10 % by volume or more, more preferably 15 % by volume or more, even more preferably 20 % by volume or more, even more preferably at least 30 % by volume, particularly preferably 50 % by volume or more, and most preferably from 75 % by volume or more, with respect to 100 % by volume of the raw material gas. When two or more different alcohols are used in combination, the sum of the amounts thereof is preferably within in the above range. The use of the catalyst according to the present invention enables the reaction to proceed efficiently even when the alcohol concentration in the raw material gas is high.

(Aldehyde)

Aldehydes are usually oxides of alcohols. Specific examples thereof include formaldehyde, acetaldehyde, propionaldehyde, butyraldehyde, valeraldehyde, and the like.

When the raw material gas contains an aldehyde, the aldehyde is generally one corresponding to the alcohol. Specifically, when methanol is used as the alcohol, the aldehyde is formaldehyde. Likewise, the aldehyde is acetaldehyde when using ethanol as the alcohol, the aldehyde is propionaldehyde when using propanol as the alcohol, the aldehyde is butyraldehyde when using butanol as the alcohol, and the aldehyde is valeraldehyde when using pentanol as the alcohol. However, the aldehyde may include an aldehyde other than the aldehyde corresponding to the alcohol.

The concentration of the aldehyde in the raw material gas is preferably 1% by volume or more, more preferably 5% by volume or more, even more preferably 10% by volume or more, even more preferably 30% by volume or more, particularly preferably 50% by volume or more, and most preferably from 75 to 99 % by volume, with respect to 100 % by volume of the raw material gas. When two or more different alcohols are used in combination, the sum of the amounts thereof is preferably within in the above range.

The total concentration of the alcohol and the aldehyde in the raw material gas is preferably 15% by volume or more, more preferably 20% by volume or more, and even more preferably from 20 to 40% by volume, with respect to 100 % by volume of the raw material gas.

(Inert gas)

Examples of the inert gas include, but are not particularly limited, nitrogen gas and argon gas. One of these inert gases may be used alone or two or more of these may be used in combination.

The concentration of the inert gas is preferably 90% by volume or less, more preferably 30 to 90% by volume, even more preferably 50 to90 % by volume, and particularly preferably 60 to80 % by volume, with respect to 100 % by volume of the raw material gas.

(Contact)

With respect to the method for bringing the raw material gas into contact with the catalyst, there is no particular limitation. A preferred example thereof is a method in which the raw material gas is passed through the reaction bed in the reaction tube so as to allow the synthesis catalyst in the reaction bed to contact the raw material gas.

The temperature (reaction temperature) at which the catalyst is brought into contact with the raw material gas is preferably from 300 to 500 °C, more preferably from 350 to 450 °C. The reaction temperature of not lower than 300 °C is preferable in that the reaction rate is sufficiently increased, and the diene compound can be produced more efficiently. On the other hand, the reaction temperature of not higher than 500 °C is preferable in that deterioration of the catalyst can be prevented or suppressed.

The pressure (reaction pressure) at which the raw material gas is brought into contact with the catalyst is preferably 0.1 to 10 MPa, and more preferably 0.1 to 3 MPa. The reaction pressure of not less than 0.1 MPa is preferable in that the reaction rate is increased, and the diene compound can be produced more efficiently. On the other hand, the reaction pressure of not more than 10 MPa is preferable in that deterioration of the catalyst can be prevented or suppressed.

The space velocity (SV) of the raw material gas in the reaction bed is usually adjusted appropriately in consideration of the reaction pressure and the reaction temperature, but is preferably 0.1 to 10000 L/h/L-catalyst, more preferably 10 to 5000 L/h/L-catalyst, and even more preferably 100 to 2500 L/h/L-catalyst, in terms of the value under the standard condition. The space velocity is appropriately adjusted in consideration of the reaction pressure and the reaction temperature.

For example, when butadiene is produced using the production apparatus 10, the temperature controller 5 and the pressure controller 6 adjust the internal temperature and pressure of the reaction tube 1 to the respective predetermined values. The raw material gas 20 is supplied from the supply pipe 3 into the reaction tube 1. In the reaction tube 1, the raw material comes into contact with the synthesis catalyst and reacts to generate butadiene. The product gas 22 containing butadiene is released from the outlet pipe 4. The product gas 22 may contain compounds such as acetaldehyde, propylene, and ethylene.

With respect to the product gas 22 containing the diene compound, the product gas is subjected to purification such as gas-liquid separation or distillation purification as necessary to remove unreacted raw materials and by-products.

The present invention also enables the production of a diene compound from bioethanol to thereby reduce the environmental burden.

[Examples]

Hereinbelow, the present invention will be described with reference to Examples which, however, should not be construed as limiting the present invention.

[Characterization of carrier and catalyst]

1. Measurement of BET Specific Surface Area

BET specific surface area was measured using the product name Gemini2375 manufactured by Micromeritics after drying 50 mg of the carrier or the catalyst at 120°C for 12 hours in a vacuum atmosphere.

Production Example 1)

In "Catalyst composition" in Table 1, the numerical value next to each element symbol indicates the amount of the element supported on the carrier (% by mass) relative to 100 % by mass of the porous carrier. For example, the indication "Hf3Zn0.5Cul" for Production Example 1 means that 3% by mass of Hf, 0.5% by mass of Zn, and 1% by mass of Cu were supported in terms of the amounts of respective elements, each with respect to 100% by mass of the porous carrier.

Hafnium chloride (IV) was dissolved in water to obtain a first impregnation solution. The first impregnation was carried out by dropping this impregnation solution on a porous silica carrier (product name: CARiACT Q-6, manufactured by Fuji Silysia chemical LTD., particle diameter: 1.18 to 2.36 mm, total pore volume: 0.61 mL/g, specific surface area: 430 m$^2$/g, average pore diameter: 5.7 nm) so that the supported amount of Hf would meet the composition shown in Table 1 and the pore filling ratio of the impregnation solution would be 100%. The porous silica carrier after the first impregnation was dried at 110°C for 3 hours in air and then calcined at 400°C for 4.5 hours to obtain a catalyst precursor in which the Hf compound was supported on the porous silica carrier.

Zinc nitrate hexahydrate and copper nitrate trihydrate were dissolved in water to obtain a second impregnation solution. The second impregnation was carried out by dropping the second impregnation solution on the catalyst precursor such that the supported amounts of Zn and Cu would meet the composition shown in Table 1 and the pore filling ratio of the impregnation solution would be 100%. The total pore volume of the porous silica support was regarded as the total pore volume of the catalyst precursor. The catalyst precursor after the second impregnation was dried in air at 110°C for 3 hours and then calcined at 400°C for 4.5 hours to obtain a butadiene synthesis catalyst in which the Hf, Zn, and Cu compounds were supported on a porous silica support.

(Production Example 2)

The butadiene synthesis catalyst was obtained in the same manner as in Production Example 1 except for the use of a porous silica carrier (product name "CARiACT Q-10" manufactured by Fuji Silysia chemical LTD., particle diameter: 1.18 to 2.36 mm, total pore volume: 0.95 mL/g, specific surface area: 300 m$^2$/g, average pore diameter: 12.7 nm).

(Production Example 3)

The butadiene synthesis catalyst was obtained in the same manner as in Production Example 1 except for the use of a porous silica carrier (product name "CARiACT Q-15" manufactured by Fuji Silysia chemical LTD., particle diameter: 1.18 to 2.36 mm, total pore volume: 0.99 mL/g, specific surface area: 195 m$^2$/g, average pore diameter: 20.3 nm).

(Production Example 4)

The butadiene synthesis catalyst was obtained in the same manner as in Production Example 1 except for the use of a porous silica carrier (product name "CARiACT Q-30" manufactured by Fuji Silysia chemical LTD., particle diameter: 1.18 to 2.36 mm, total pore volume: 0.98 mL/g, specific surface area: 107 m$^2$/g, average pore diameter: 36.6 nm).

Table 1 shows the physical properties of the porous silica supports used in Production Examples 1 to 4 and the obtained butadiene synthesis catalysts. The average pore diameter of the porous support and the butadiene synthesis catalyst was calculated from the total pore volume V1 and the specific surface area A1 using formula (II) above.

[Table 1]

| | | | Production Example 1 | Production Example 2 | Production Example 3 | Production Example 4 |
|---|---|---|---|---|---|---|
| Porous Carrier | Specific Surface Area | m$^2$/g | 430 | 300 | 195 | 107 |
| | Pore Volume | mL/g | 0.61 | 0.95 | 0.99 | 0.98 |
| | Average Pore Diameter | nm | 5.7 | 12.7 | 20.3 | 36.6 |

(continued)

| Butadiene Synthesis Catalyst | | | Production Example 1 | Production Example 2 | Production Example 3 | Production Example 4 |
|---|---|---|---|---|---|---|
| | Catalyst composition | | Hf3Zn0.5Cu1 | Hf3Zn0.5Cu1 | Hf3Zn0.5Cu1 | Hf3Zn0.5Cu1 |
| | Specific Surface Area | m²/g | 350 | 265 | 170 | 100 |
| | Pore Volume | mL/g | 0.57 | 0.9 | 0.87 | 0.79 |
| | Average Pore Diameter | nm | 6.5 | 13.6 | 20.5 | 31.4 |

(Evaluation Method)

3.4 g of the each catalyst of Production Examples 1 to 4 was filled into a cylindrical reaction tube made of stainless steel and having a diameter of 1/2 inch (1.27 cm), and a length of 15.7 inch (40 cm) to form a reaction bed.

Next, the reaction temperature (reaction bed temperature) was set to 400°C , the reaction pressure (reaction bed pressure) was set to 0.1 MPa, and a raw material gas was supplied to the reaction tube at an SV of 1275 L/hr/L-catalyst to obtain a product gas. The raw material gas was a mixed gas of 30 % by volume (in terms of gas volume) of ethanol and 70 % by volume (in terms of gas volume) of nitrogen. The recovered product gas was analyzed by gas chromatography, and the selectivities for butadiene (1,3-butadiene), acetaldehyde, ethylene and propylene, the conversion of the raw material, and the butadiene yield were determined. The butadiene yield is a value determined by [conversion of raw material] $\times$ [selectivity for butadiene].

[Examples 1 to 4]

The butadiene synthesis reaction was carried out using the butadiene synthesis catalysts obtained in Production Examples 1 to 4. These results are shown in Table 2.

[Table 2]

| | Catalyst | Conversion of Raw Material (mol%) | Selectivity (mol%) | | | | | Butadiene Yield (mol%) |
|---|---|---|---|---|---|---|---|---|
| | | | Butadiene | Acetaldehyde | Ethylene | Propylene | Other | |
| Example 1 | Production Example 1 | 93.8 | 52.0 | 5.0 | 10.3 | 4.1 | 28.6 | 48.8 |
| Example 2 | Production Example 2 | 96.4 | 56.9 | 4.9 | 7.3 | 3.9 | 27.0 | 54.9 |
| Example 3 | Production Example 3 | 89.7 | 50.1 | 12.5 | 6.5 | 3.1 | 27.8 | 44.9 |
| Example 4 | Production Example 4 | 88.9 | 48.8 | 16.1 | 4.8 | 2.9 | 27.4 | 43.4 |

From the results in Table 2, it can be seen that butadiene can be obtained suitably in Examples 1 to 4 even when the raw material gas contains a large amount of ethanol.

[Reference Signs List]

1    Reaction tube
2    Reaction bed
3    Supply pipe
4    Outlet pipe
5    Temperature controller
6    Pressure controller
10   Butadiene production apparatus

**Claims**

1.  A catalyst comprising, as catalyst components: a compound comprising at least one element X selected from the group consisting of elements belonging to Groups 3 to 6 of the periodic table; a zinc (Zn) compound; and a compound comprising at least one element Y selected from the group consisting of elements belonging to Groups 7 to 11 of the periodic table,
    wherein the catalyst has an average pore diameter of 2 to 50 nm.

2.  The catalyst according to claim 1, wherein the catalyst components are supported on a porous carrier.

3.  The catalyst according to claim 2, wherein the porous carrier has mesopores.

4.  The catalyst according to any one of claims 1 to 3, wherein a specific surface area of the catalyst is 50 to 1000 $m^2/g$.

5.  The catalyst according to any one of claims 1 to 4, which is a catalyst for synthesizing a diene compound from a raw material gas containing an alcohol.

6.  The catalyst according to claim 5, wherein the raw material gas comprises ethanol, or a mixture of ethanol and acetaldehyde.

7.  A method for producing a diene compound, comprising contacting the catalyst of any one of claims 1 to 6 with a raw material gas containing an alcohol to obtain a diene compound.

8.  The method according to Claim 7, wherein an amount of the alcohol is 10 % by volume or more with respect to 100 % by volume of the raw material gas.

FIG. 1

## INTERNATIONAL SEARCH REPORT

| | International application No. |
|---|---|
| | PCT/JP2019/001294 |

**A. CLASSIFICATION OF SUBJECT MATTER**
Int.Cl. B01J23/80(2006.01)i, C07C1/24(2006.01)i, C07C11/167(2006.01)i,
C07B61/00(2006.01)n, C07C11/04(2006.01)n, C07C11/06(2006.01)n,
C07C45/29(2006.01)n, C07C47/07(2006.01)n
According to International Patent Classification (IPC) or to both national classification and IPC

**B. FIELDS SEARCHED**

Minimum documentation searched (classification system followed by classification symbols)
Int.Cl. B01J23/80, C07C1/24, C07C11/167, C07B61/00, C07C11/04,
C07C11/06, C07C45/29, C07C47/07

| Documentation searched other than minimum documentation to the extent that such documents are included in the fields searched | |
|---|---|
| Published examined utility model applications of Japan | 1922–1996 |
| Published unexamined utility model applications of Japan | 1971–2019 |
| Registered utility model specifications of Japan | 1996–2019 |
| Published registered utility model applications of Japan | 1994–2019 |

Electronic data base consulted during the international search (name of data base and, where practicable, search terms used)
JSTPlus/JST7580 (JDreamIII)

**C. DOCUMENTS CONSIDERED TO BE RELEVANT**

| Category* | Citation of document, with indication, where appropriate, of the relevant passages | Relevant to claim No. |
|---|---|---|
| X | WO 2016/043209 A1 (SEKISUI CHEMICAL CO., LTD.) 24 March 2016, claims 1, 5, paragraphs [0080], [0081] & US 2017/0260112 A1, claims 1, 5, paragraphs [0160]-[0167] & EP 3196181 A1 | 1-8 |
| X | JP 2017-144421 A (SEKISUI CHEMICAL CO., LTD.) 24 August 2017, paragraphs [0053]-[0057] (Family: none) | 1-8 |

☒ Further documents are listed in the continuation of Box C. ☐ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 11 March 2019 (11.03.2019) | 26 March 2019 (26.03.2019) |

| Name and mailing address of the ISA/ | Authorized officer |
|---|---|
| Japan Patent Office 3-4-3, Kasumigaseki, Chiyoda-ku, Tokyo 100-8915, Japan | |
| | Telephone No. |

Form PCT/ISA/210 (second sheet) (January 2015)

**INTERNATIONAL SEARCH REPORT**

| International application No. |
|---|
| PCT/JP2019/001294 |

C (Continuation).   DOCUMENTS CONSIDERED TO BE RELEVANT

| Category* | Citation of document, with indication, where appropriate, of the relevant passages | Relevant to claim No. |
|---|---|---|
| X | JP 2016-23141 A (BRIDGESTONE CORPORATION) 08 February 2016, paragraphs [0023], [0026], [0030], [0031], [0036] (Family: none) | 1-8 |
| X | CN 105413695 A (INSTITUTE OF COAL CHEMISTRY, CHINESE ACADEMY OF SCIENCES) 23 March 2016, claim 1, paragraphs [0125]-[0128], [0140]-[0144] (Family: none) | 1, 4-8 |
| X | WO 2014/097942 A1 (SEKISUI CHEMICAL CO., LTD.) 26 June 2014, paragraphs [0068], [0069], [0071], [0072], [0083] & US 2015/0284306 A1, paragraphs [0126], [0127], [0129]-[0135], [0154]-[0156] & EP 2937142 A1 & CN 104870088 A | 1-4 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2018005943 A
- WO 2014199349 A